# EUROPEAN PATENT SPECIFICATION

(11) **EP 0 957 111 B1**
(45) Date of publication and mention of the grant of the patent: **29.12.2004**
(21) Application number: 99107035.0
(22) Date of filing: 09.04.1999
(51) Int. Cl.: C07K 7/06, C07K 7/08, C07K 16/06, A61K 39/395, C07K 16/28

(54) **Specific binding proteins for treating canine allergy**
Spezifische Bindungsproteine zur Behandlung von Allergien beim Hund
Protéines de liaison spécifiques pour le traitement des allergies canines

(30) Priority: 09.04.1998 US 58331; 30.03.1999 US 281760
(43) Date of publication of application: 17.11.1999
(73) Proprietor: IDEXX LABORATORIES, INC., Westbrook, ME 04092 (US)
(72) Inventor: Lawton, Robert, Gorham, Maine 04938 (US); Mermer, Brion, Cumberland, Maine 04092 (US); Francoeur, Greg, North Yarmouth, Maine 04097 (US)
(74) Representative: Viering, Jentschura & Partner

(56) References cited:
- D.DEBOER ET AL.: "Production and characterization of mouse monoclonal antibodies directed againast canine IgE and IgG" VETERINARY IMMUNOLOGY AND IMMUNOPATHOLOGY, vol. 37, 1993, pages 183-99, XP000647974
- KONIECZNY A. ET AL.: "The major dog allergens, Can f1 and Can f2, are silvary lipocalin proteins: cloning and immunological characterization of the recombinant forms" IMMUNOLOGY, vol. 92, 1997, pages 577-86, XP000915647

## Description

### FIELD OF THE INVENTION

This invention concerns peptides. More particularly, the invention concerns compositions for administration to dogs, which actively provide immunity to the dog's immunoglobulin E molecules.

### BACKGROUND ART

It is estimated that up to 30% of all dogs suffer from allergies or allergy-related skin disorders. Specifically, allergic dermatitis has been estimated to affect between 3 and 15% of the entire canine population. Given the prevalence of allergies in dogs, there is a need to develop methods and compositions to properly diagnose and treat canine allergies.

The substances most likely to cause an allergic reaction vary from species to species. Common canine allergens include fleas, pollens, molds and dust. Allergy to fleas is believed to be the most common dog allergy. Typically, a flea's saliva is the allergen, and a single fleabite can cause substantial itching. An additional form of allergy in dogs is termed atopy. Atopy is a condition where a dog is allergic to inhalants such as pollens, molds or microscopic mites such as are found in house dust.

Antibody molecules play a role in allergic manifestations. In mammals, antibody molecules are classified into various isotypes referred to as IgA, IgD, IgE, IgG, and IgM. Antibody molecules consist of both heavy and light chain components. The heavy chains of molecules of a given isotype have extensive regions of amino acid sequence homology, and conversely have regions of difference from antibodies belonging to other isotypes. The shared regions of the heavy chains provide members of each isotype with common abilities to bind to certain cell surface receptors or to other macromolecules, such as complement. These heavy chain regions, therefore, serve to activate particular immune effector functions. Accordingly, separation of antibody molecules into isotypes serves to separate the antibodies according to a set of effector functions that they commonly activate.

In humans and dogs, immunoglobulin E (hereinafter IgE) is involved in allergy. Thus, IgE is the antibody type that is understood to be an important mediator of allergic responses, including Type I immediate hypersensitivity.

IgE molecules bind to mast cells and basophils. This binding occurs when the Fc region of the IgE molecule is bound to Fc receptors on the mast cells. When such bound IgE antibodies then bind to an allergen, the allergen cross-links multiple IgE antibodies on the cell surface. This cross-linking mediates Type I immediate hypersensitivity reactions and causes release of histamines and other molecules that produce symptoms associated with allergy.

Monoclonal antibodies having different degrees of sensitivity to canine IgE and IgG have been identified. (DeBoer, et al. Immunology and Immunopathology 37, 183-199 (1993).) DeBoer, et al. identified several monoclonal antibodies which had cross reactivity between IgG and IgE. (See, e.g., DeBoer, et al., Table 4 and accompanying text.) Three monoclonal antibodies (A5, D9, and B3) were identified by DeBoer et al., as having some affinity for canine IgE. Of the monoclonal antibodies identified in DeBoer et al., antibody D9 appeared to have the greatest degree of neutralization of Prausnitz-Kustner reactivity for atopic dog serum. In the context of canine allergy, DeBoer et al. proposed use of their monoclonal antibodies (MAbs) in the use of antigen-specific IgE ELISA, and for quantifying canine IgE. Additionally, they proposed use of their MAbs for immunostaining of Western Blot assays, to evaluate the molecular specificity of IgE antibodies, as well as for *in vitro* studies on degranulation of mast cells.

In humans the serum level of total IgE is diagnostic of allergic disease. To explore the possibility that the serum level of IgE might also be diagnostic of allergy in dogs, several studies were performed. (Hill and DeBoer Am. J. Yet. Res., (July 1994) *55*(*7*), 944-48). Publications following the DeBoer article used a monoclonal antibody designated D9 in an ELISA assay having the following configuration: D9 was bound to a substrate, antibodies were captured by D9 and then D9 having a marker was used to flag the captured antibody. The Hill and DeBoer ELISA was used to establish the total amount of IgE in canine serum in an effort to diagnose canine allergy. In contrast to humans, the quantity of IgE determined to exist in canine circulation was of no use whatsoever in the diagnosis of allergy in dogs. (See, e.g., Abstract and Discussion Sections of Hill and DeBoer) This finding was in direct contrast to the situation in human immunology.

This divergent diagnostic result based on levels of IgE in humans compared to such levels in dogs, points out the difficulty of any attempt to correlate data between animals of two different genera. This difficulty is further exacerbated by the fact that dogs can be allergic to a different set of antigens than humans are. Fleas, for instance, are a severe problem for dogs, but not humans. Furthermore, in instances where dogs and humans appear to be allergic to the same allergen extract, studies by doctors Esch and Greer of Greer Laboratories, have indicated that the specific allergens in an allergen extract which produce canine disease are not necessarily the same allergens that produce disease in humans. For example, it is known that the immunodominant components of dust mite extracts are different in dogs than in humans.

The genomic sequences encoding human and murine IgE heavy chain constant region are known (For example, see Ishida et al., "The Nucleotide Sequence of the Mouse Immunoglobulin E Gene: Comparison with the Human Epsilon Gene Sequence", EMBO Journal *1*,1117-1123 (1982). A comparison of the human and murine genes shows that they possess 60% homology within exons, and 45-50% homology within introns, with various insertions and deletions.

Patel et al. published the nucleotide and predicted amino acid sequence for exons 1-4 of the heavy chain constant region of canine IgE in the article entitled "Sequence of the Dog Immunoglobulin Alpha and Epsilon Constant Region Genes," Immunogenetics *41*, 282-286 (March 22, 1995). The complete sequence of the canine IgE heavy chain constant region, with membrane bound portions encoded by exons 5 and 6 are disclosed in copending applications Serial Numbers 08/800,698 filed February 14, 1997, 09/146,400 filed September 3, 1998, and 09/146,617 filed September 3, 1998.

Because IgE is believed to mediate allergic symptoms, it may be desirable to decrease IgE levels as a mechanism for alleviating allergic symptoms. However, a patient's own IgE molecules are self-proteins, and immune responses to such proteins are usually suppressed. The suppression of immune responses to self-proteins, i.e., tolerance to self-antigens, is hypothesized to occur in a number of ways.

The current hypothesis for suppression of T cells directed to self-antigens, involves an induction of "clonal deletion" of such T cells in the thymus, whereby T cell receptors which might recognize self-peptides in association with MHC molecules are eliminated, and only those which recognize foreign peptide and MHC molecules are allowed to expand. In addition, suppressor T cells may also exist which prevent the induction of immune responses to self-proteins.

In contrast to the situation with T cells, it is believed that there are many B cells which express receptors (i.e., surface immunoglobulin) for self-proteins, and that the reason these cells do not produce antibodies to self-proteins is because the T cells required for the antigen presentation to the B cell are normally missing.

A B cell which recognizes epitopes (antigen-binding sites) on a patient's own IgE antibodies is capable of generating antibodies, generally IgG, directed to this self-antigen, i.e., IgE. The existence of such B cells, therefore, presents a unique opportunity to induce the production of auto-antibody responses. There is an unmet need for such antibodies in order to treat allergic disease.

The hypothesis regarding "antigen presentation" involves: the recognition of antigen by surface immunoglobulin on the B cell, the internalization and processing of this antigen, the association of peptides derived from the antigen with MHC molecules expressed on the surface of the B cell, and then, the recognition of the associated antigen peptide and MHC molecules by a particular T cell. The T-cell:B-cell interaction then leads to signal transduction in both cells and the synthesis and elaboration of soluble cytokines which eventually result in antibody production by the B cell.

Thus, in most circumstances, only when an antigen is foreign does an immune response occur; otherwise the internalization and processing of self-proteins would regularly lead to the presentation of self peptide-MHC complexes to T cells and thereby lead to autoimmune antibodies.

Therefore, in order to induce an antibody response to a self-peptide, such as IgE, the immune system must be manipulated so as to allow an auto-reactive B cell to become an antibody-secreting B cell. There is an unmet need to manipulate the immune system in this way, particularly in the context of allergic disease.

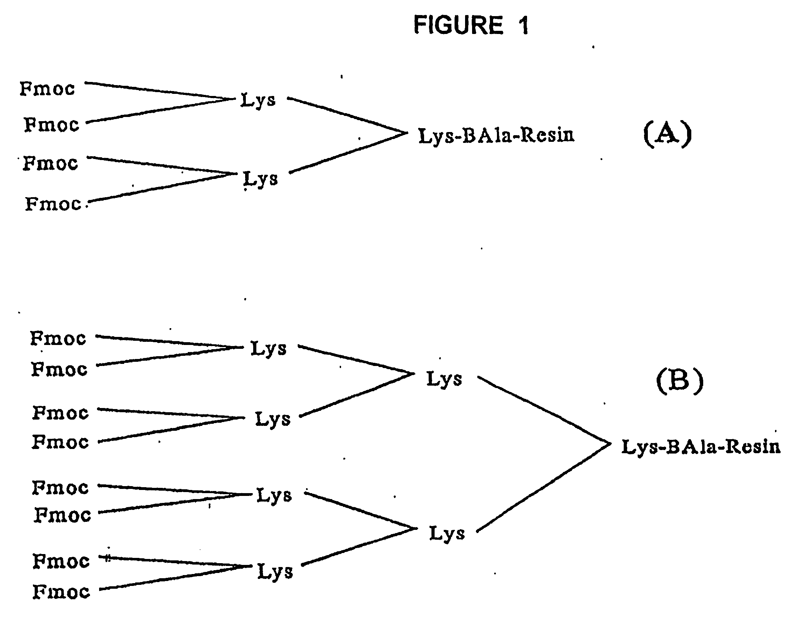

In general, there are several known approaches for generating antibodies to peptide antigens. For example, multiple antigenic peptides (MAPs), introduced by Dr. James Tam (Tam, J.P., (1988) Proc. Natl. Acad. Sci. U.S.A. *85*, 5409-5413), have demonstrated several advantages for inducing anti-peptide antibodies. The MAP approach is an improved alternative to the conventional technique of conjugating a peptide antigen to a protein carrier. One of the primary limitations associated with the use of protein carriers is the large mass of the carrier relative to the attached peptide antigen. This relative size disparity may result in a low ratio of anti-peptide antibodies compared to anti-carrier antibodies. MAPs typically have 4 or 8 peptide arms branching out from a lysine core matrix as depicted in Fig.1 A-B. The peptide antigen is conjugated to each arm. Thus there is a much higher ratio of antigen to carrier molecule in a MAP system compared to traditional protein conjugation. This design maximizes the concentration of the antigen for a specific immunogenic response. Moreover, the central lysine core of the MAP-peptide has been shown to be non-immunogenic. (Tam, J.P., Proc. Natl. Acad. Sci. U.S.A. *85*, 5409-5413 (1988); Posnett, D.N., McGrath, H., and Tam, J.P., J. Biol. Chem. *263*, 1719-1725 (1988)) Therefore, antibodies induced to MAP-peptides are a direct response to the antigen. Accurate knowledge of the chemical composition, structure, and quantity of the peptide prior to immunization is possible by directly synthesizing the antigen onto the branching lysine core. Also, because the MAP approach removes the need to conjugate peptides to carrier proteins, which may alter the antigenic determinants, chemical ambiguity is eliminated. Thus MAPs are believed to induce antibody responses of high purity, increased avidity, accurate chemical definition, and improved safety.

Fmoc MAP resins (available from Applied Biosystems, Foster City, Calif.) are Fmoc-compatible resins connected to a small core matrix of branching lysine residues. The core matrix comprises several levels of lysine residues attached to the previous lysine at both the N-α and N-ε amino groups, as depicted in Figure 1A-B.

MAP-peptides used in experimental vaccine design have elicited high titers of anti-peptide antibodies that recognize the native protein. (Tam, J.P., (1988) Proc. Natl. Acad. Sci. U.S.A. *85*, 5409-5413; Posnett, D.N., McGrath, H., and Tam, J.P., (1988) J. Biol. Chem. *263*, 1719-1725; Auriault, C., Wolowczuk, I., Gras-Masse, H., Maguerite, M., Boulanger, D., Capron, A., and Tartar, A., (1991) Peptide Res.*4*, 6-11). Additionally, increased sensitivity and reliability of antibody-antigen interactions in solid-phase immunoassays have been observed with MAP-peptides due to enhanced coating capacity and avidity. (Tam, J.P., and Zavala, F., (1989) J. Immunol. Meth., 124, 53-61).

An additional approach that is known to be useful for generating antibodies to peptide antigens involves placing multiple copies of peptides on the surface of plant virus particles. EPICOAT™ technology (Axis Genetics plc, Cambridge, England) is one such example. The EPICOAT™ technology is based on chimeric virus particle (CVP) technology that utilizes the recombinant genetic modification of plant viruses.

The EPICOAT™ technology involves insertion of a small portion of a foreign protein (a peptide) into a plant virus in such a way that multiple copies of the peptide are displayed on the surface of the virus particle. The EPICOAT™ technology is currently based on the cow pea mosaic virus (CPMV) a plant virus that infects the cow pea plant, also known as the "black-eyed" bean. The unmodified CPMV particle is icosahedral, and about twenty-eight nanometers (nm.) in diameter. CPMV particles are composed of two proteins, referred to as the large and small coat proteins. Studies have revealed a site within the small coat protein which allows presentation of a foreign peptide in a prominent position on the virus surface, whereby up to sixty copies of a particular peptide can be presented on each virus particle.

With the EPICOAT™ technology, DNA copies of the plant virus's genetic material are used. A minute quantity of the DNA encoding the virus protein, including the inserted foreign peptide, is applied to the leaves of young cow pea plant, along with an abrasive powder. Upon gentle rubbing, DNA enters the leaves and utilizes the plant's own cellular mechanisms to initiate generation of functional virus particles. The virus replicates within the inoculated leaves and spreads throughout the growing plant.

After two to three weeks, leaf material containing large quantities of the virus is harvested. Chimeric virus particles (CVPs) are isolated by centrifugation and selective precipitation of homogenized plant material. Between 1 to 2 grams of CVPs can be obtained per kilogram fresh weight of leaf material. Cow pea plants are readily grown in abundance in controlled environments, allowing generation of large quantities of CVPs.

It has been reported that peptides of up to thirty-six amino acids in size have been successfully incorporated into CVPs. The resulting particles are extremely robust with a thermal inactivation point of 65°C. The CVPs have been shown to withstand acidic pH as well as protein degrading enzymes. It has been reported that the expressed peptides are capable . of eliciting specific immune responses in animals. It has been hypothesized that surface presentation of peptides may enhance the recognition by the host immune system, and may provide a route for development of recombinant sub-unit vaccines and immuno-therapeutics.

DeBoer et al., 1993 (Veterinary Immunology and Immunopathology, 37 (1993) 183-199) have prepared a series of murine hybridoma cell lines that produced monoclonal antibodies with specificity for canine immunoglobuline E and G. These monoclonal antibodies were tested for their ability to induce a reverser cutaneous anaphylaxis reaction in dog skin, to neutralize the Pransnitz-Küstner reactivity of atopic dog serum, to serve as a ligand in immunoaffinity chromatography, and to bind to IgE and other Ig subclasses in several ELISA systems. Some of these monoclonal antibodies produced were found to be specific for canine IgE.

Konieczny et al., 1997 (Immunology 1997, 92, 577-586) report the isolation of cDNAs encoding major allergene proteins and present their nucleotide and deduced amino acid sequences. Both proteins (VEG proteins and MUP) are members of the lipocaline family of small ligand-binding proteins. Recombinant forms of these proteins were produced and tested for immunoglobuline E reactivity. In addition, both recombinant proteins were able to cross-link IgE and elicit histamine released from peripheral blood leucozytes in vitro.

Burt et al., 1987 (Molecular Immunology, Vol. 24, No. 4, pp. 379-389, 1987) describe the production of polyclonal antisera with pre-determined specificities for a range of rat IgE epitopes by immunizing rabbits KLH-conjugates of five different synthetic peptides representing different sequences in the CH3 and CH4 domain of rat IgE. The anti-peptide sera were tested in functional assays designed to investigate the mode of interaction between rat IgE and its receptor on rat mast cells. Each anti-peptide serum was capable of inhibiting the binding of IgE to mast cells and furthermore, able to initiate the secretion of histamine from cells sensitised with rat IgE in an "anti-IgE" induced manner.

Presta et al., 1993 (Journal of Immunology, Vol. 151,2623-2632, No. 5,1993) describes the humanization of a murine antibody with the ability to block binding of IgE to its high-affinity receptor but which does not bind to IgE once it is bound to the receptor, because this would trigger histamine release. Variants of the humanized antibody were evaluated to probe the importance of framework residues on antibody binding and to determine which charged residues in the CDR interacted with IgE. It was found that only five changes in human framework residues were required to provide for binding comparable to that of the original murine antibody.

### DISCLOSURE OF THE INVENTION

Disclosed is a specific binding protein which specifically binds to native canine free or B cell-bound IgE exon 3, and which does not bind to IgE exon 3 when the IgE is bound to receptor on a mast cell. The surface-bound IgE can be IgE expressed on the surface of a canine B cell.

Disclosed is a specific binding protein which specifically binds to an isolated and purified peptide comprising a leucine positioned two peptide bonds away from a tyrosine-arginine pair; e.g., SEQ ID NO:1 leucine-blank-blank-tyrosine-arginine, SEQ ID NO:2 tyrosine-arginine-blank-blank-leucine, or SEQ ID NO:3 leucine-blank-blank-tyrosine-arginine-blank-blank-leucine. The peptide can consist of from 5 to 71 amino acids.

Disclosed is an antibody which binds to a defined epitope and which is raised to an isolated and purified peptide comprising an amino acid sequence, or a conservative variant thereof, which comprises: SEQ ID NO:4 Thr-Leu-Leu-Glu-Tyr-Arg-Met; also disclosed is a recombinant binding molecule which specifically binds to the defined epitope bound by the antibody.

Disclosed is an antibody which binds to a defined epitope and which is raised to an isolated and purified peptide comprising an amino acid sequence, or a conservative variant thereof, which comprises: SEQ ID NO:5 Gly-Met-Asn-Leu-Thr-TrpTyr-Arg-Glu-Ser-Lys; also disclosed is a recombinant binding molecule which specifically binds to the defined epitope bound by the antibody.

Also disclosed is a specific binding protein which is raised to a multiply antigenic peptide comprising multiple copies of an isolated and purified peptide which comprises a leucine positioned two peptide bonds away from a tyrosine-arginine pair; the specific binding protein specifically binds to a defined epitope. The isolated and purified peptide can be from 5-71 amino acids. Also disclosed is a recombinant binding molecule which specifically binds to the defined epitope bound by the binding protein.

Disclosed is a specific binding protein which specifically binds to an isolated and purified peptide comprising a cysteine positioned two peptide bonds away from a proline-histidine pair positioned three peptide bonds from a cysteine. The peptide can have the form: SEQ ID NO:6 cysteine-blank-blank-proline-histidine-blank-blank-blank-cysteine. The cysteines may form a covalent bond through a reduction reaction, cyclizing the peptide and becoming cystine. The peptide can comprise from 9 to 71 amino acids.

Disclosed is an antibody that binds to a defined epitope and which is raised to an isolated and purified peptide that has the amino acid sequence, or a conservative variant thereof, which comprises: SEQ ID NO:7 serine-valine-threonine-leucine-cysteine-proline-asparagine-proline-histidine-isoleucine-proline-methionine-cysteine-glycine-glycine-glycine. The cysteines may form a covalent bond through a reduction reaction, cyclizing the peptide and becoming cystine. Also disclosed is a recombinant binding molecule that specifically binds to the defined epitope bound by the antibody.

Disclosed is an antibody that binds to a defined epitope and which is raised to an isolated and purified peptide that has the amino acid sequence, or a conservative variant thereof, which comprises: SEQ ID NO:8 serine-alanine-cysteine-proline-asparagine-proline-histidine-asparagine-proline-tyrosine-cysteine-glycine-glycine-glycine. The cysteines may form a covalent bond through a reduction reaction, cyclizing the peptide changing the amino acid to cystine. Also disclosed is a recombinant binding molecule that specifically binds to the defined epitope bound by the antibody.

Disclosed is a specific binding protein that specifically binds to an isolated and purified peptide comprising a cysteine positioned one peptide bond from a proline-histidine pair, positioned one peptide bond from a proline, positioned two peptide bonds from a cysteine; the peptide can have the form: SEQ ID NO:9 cysteine-blank-proline-histidine-blank-proline-blank-blank-cysteine. The cysteines may form a covalent bond through a reduction reaction, cyclizing the peptide and becoming cystine. The peptide can comprise from 9 to 71 amino acids.

Disclosed is an antibody that binds to a defined epitope and which is raised to an isolated and purified peptide that has the amino acid sequence, or a conservative variant thereof, which comprises: SEQ ID NO:10 serine-alanine-cysteine-histidine-proline-histidine-leucine-proline-lysine-serine-cysteine-glycine-glycine-glycine. The cysteines may form a covalent bond through a reduction reaction, cyclizing the peptide and becoming cystine. Also disclosed is a recombinant binding molecule that specifically binds to the defined epitope bound by the antibody.

Specific binding proteins in accordance with the invention can be antibodies, either polyclonal or monoclonal. For example the monoclonal antibody can be 8H.8 or 15A.2. The antibody in accordance with the invention will bind a defined epitope; also disclosed are recombinant binding molecules which specifically binds to the defined epitope bound by an antibody in accordance with the invention.

Also disclosed is the use of a specific binding protein in accordance with the invention for the manufacture of a pharmaceutical composition for treatment or prophylaxis for canine allergy. In a preferred use, this pharmaceutical composition further comprises a diluent. Further disclosed is the use of a specific binding protein for the manufacture of a pharmaceutical composition kit, that kit comprising first dose of said specific binding protein and booster dose of said specific binding protein.

| **AMINO ACID ABBREVIATIONS** | | |
|---|---|---|
| **Amino Acid** | **One-Letter Symbol** | **Three-Letter Symol** |
| alanine | A | ala |
| arginine | R | arg |
| asparagine | N | asn |
| aspartic acid | D | asp |
| cysteine | C | cys |
| glutamic acid | E | glu |
| glutamine | Q | gln |
| glycine | G | gly |
| histidine | H | his |
| isoleucine | I | ile |
| leucine | L | leu |
| lysine | K | lys |
| methionine | M | met |
| phenylalanine | F | phe |
| proline | P | pro |
| serine | S | ser |
| threonine | T | thr |
| tryptophan | W | trp |
| tyrosine | Y | tyr |
| valine | V | val |

*cDNA* clone: A duplex DNA sequence representing an RNA, carried in a cloning vector.

*Cloning*: The selection and propagation of a single DNA species.

*Cloning Vector*: A plasmid, phage DNA or other DNA sequence, able to replicate in a host cell and capable of carrying exogenously added DNA sequence for purposes of amplification or expression of the added DNA sequence.

*Codon*: A triplet of nucleotides that represents an amino acid or termination signal.

*Conservative variants*: Conservative variants of nucleotide sequences include nucleotide substitutions that do not result in changes in the amino acid sequence encoded by such nucleotides, as well as nucleotide substitutions that result in conservative amino acid substitutions, e.g., amino acid substitutions which do not substantially affect the character of the polypeptide translated from said nucleotides. For example, the character of a peptide derived from IgE is not substantially affected if the substitutions do not preclude specific binding of the peptide to canine IgE receptor or other canine IgE binding ligands.

Conservative variants of amino acid sequences include amino acid substitutions or deletions that do not substantially affect the character of the variant polypeptide relative to the starting peptide. For example, polypeptide character is not substantially affected if the substitutions or deletions do not preclude specific binding of the variant peptide to a specific binding partner of the starting peptide. The term mimotope refers to a conservative variant of an amino acid sequence, to which antibody specificity has been raised. The mimotope comprises a variant of the epitope of the starting peptide such that it is able to bind antibodies that cross-react with the original epitope.

*DNA Sequence*: A linear series of nucleotides connected one to the other by phosphodiester bonds between the 3' and 5' carbons of adjacent pentoses.

*Expression*: The process undergone by a structural gene to produce a polypeptide. It is a combination of transcription and translation.

*Expression Control Sequence*: A DNA sequence of nucleotides that controls and regulates expression of structural genes when operatively linked to those genes.

*Exon*: A contiguous region of DNA encoding a portion of a polypeptide. Reference to any exon, e.g. "DNA sequence of exon 6", refers to the complete exon or any portion thereof.

*Genome*: The entire DNA of a substance. It includes *inter alia* the structural genes encoding for the polypeptides of the substance, as well as operator, promoter and ribosome binding and interaction sequences such as the Shine-Dalgarno sequences.

*Nucleotide*: A monomeric unit of DNA or RNA consisting of a sugar moiety (pentose), a phosphate, and a nitrogenous heterocyclic base. The four DNA bases are adenine ("A"), guanine ("G"), cytosine ("C") and thymine ("T"). The four RNA bases are A, G, C and uracil ("U"). A and G are purines, and C, T, and U are pyrimidines.

*Phage or Bacteriophage*: Bacterial virus, many of which include DNA sequences encapsidated in a protein envelope or coat ("capsid").

*Plasmid*: An autonomous self-replicating extrachromosomal circular DNA.

*Polymerase Chain Reaction (PCR)*: A method of amplifying a target DNA sequence contained in a mixture of DNA sequences, by using oligonucleotide primers that flank the target DNA sequence for repeated cycles of DNA synthesis of the target DNA sequence.

*Polypeptide*: A linear series of amino acids connected one to the other by peptide bonds between the a-amino and carboxyl groups of adjacent amino acids.

*Reading Frame*: The grouping of codons during translation of mRNA into amino acid sequences. For example, the sequence GCTGGTGTAAG may be translated in three reading frames or phases, each of which affords a different amino acid sequence:

GCT GGT TGT AAG-Ala-Gly-Cys-Lys
G CTG GTT GTA AG-Leu-Val-Val
GC TGG TTG TAA A-Trp-Leu-(STOP).

*Recombinant DNA Molecule*: A hybrid DNA sequence comprising at least two nucleotide sequences, the first sequence not normally being found together in nature with the second.

*Specific binding*: Binding of one substance to another at greater binding affinity than background binding. Two substances that exhibit specific binding are referred to as specific binding partners, or as a specific binding pair. An antibody and its antigen are one example of a specific binding pair.

*Specific Binding Molecule*: A molecule that exhibits specific binding to its corresponding binding partner to form a specific binding pair. As used herein, this definition of specific binding molecule covers monoclonal and polyclonal antibodies, antigen-binding fragments of these antibodies, hybrid antibodies, single-chain antibodies, and recombinant molecules capable of specific binding to a ligand.

*Structural Gene*: A DNA sequence that encodes through its template or messenger RNA ("mRNAII) a sequence of amino acids characteristic of a specific polypeptide.

*Transcription*: Synthesis of RNA on a DNA template.

*Translation*: Synthesis of peptides on the mRNA template.

### DESCRIPTION OF FIGURES

Fig. 1A depicts the core structure of a MAP protein with four arms, i.e., a 4-MAP protein; Fig. 1B depicts the core structure of a MAP protein with eight arms, i.e., an 8-MAP protein.
Fig.2 depicts data comparing the binding characteristics of several monoclonal antibodies to surface bound IgE (e.g., analogous to IgE expressed on the surface of B cells); IgE receptor (e.g., analogous to the Fc receptor on mast cells); and to a combination of IgE when bound by the IgE receptor. Thus each component assayed was immobilized on a solid surface.
Fig. 3 depicts the ability of recombinant exon 3 to inhibit conjugated antibody 8H.8 or conjugated antibody 15A.2 from binding to an IgE solid phase. Data for 8H.8 is depicted by speckled bar graphs, data for 15A.2 is depicted by solid black bar graphs.
Fig. 4 depicts the ability of soluble, purified canine IgE to inhibit monoclonal antibody 8H.8 (2.5 µg/ml) or monoclonal antibody 15A.2 (2.5 µg/ml) from binding to a canine IgE solid phase. Data for 8H.8 is depicted by speckled bar graphs; data for 15A.2 is depicted by solid black bar graphs.
Fig. 5 depicts the ability of monoclonal antibody 15A.2 or monoclonal antibody 8H.8 to inhibit IgE from binding to recombinant IgE receptor immobilized on a solid phase as detected by monoclonal antibody D9.
Fig. 6 depicts the amino acid sequences of the New England Biolabs PhDc7c phage display library that were bound by the monoclonal antibody 15A.2. These sequences are shown in alignment with the protein sequence of canine IgE.
Fig. 7 depicts the alignment of the 15A.2 binding region from seven different mammals: dog, human, green monkey, cat, swine, mouse and horse.
Fig. 8 depicts the ability of phage displaying 15A.2 mimotope peptides to inhibit canine IgE from binding the 15A.2 monoclonal antibody on the solid phase.
Fig. 9 depicts the ability of a specific 15A.2 mimotope peptide sequence attached to the solid phase to be able to bind the 15A.2 mimotope monoclonal antibody.
Fig. 10A and 10B depict the ability of a 15A.2 mimotope peptide to be able to prevent the 15A.2 monoclonal antibody from binding canine IgE on the solid phase.
Fig. 11 depicts the ability of monoclonal antibody 8H.8 and monoclonal antibody 15A.2 to bind to purified canine IgE immobilized on a solid phase.
Fig. 12 depicts data from a study evaluating the ability of antibody 15A.2 conjugated to a signal moiety to bind to solid phases having various peptides immobilized thereon.
Fig. 13 depicts data from a study evaluating the ability of monoclonal antibody 14K2, known to bind with canine IgE exon 4, to bind with various peptides immobilized on a solid phase.
Fig. 14 depicts data from a study evaluating the ability of monoclonal antibody 15A.2, known to bind to canine IgE exon 3, to bind to various peptides immobilized on a solid phase.
Fig. 15 depicts data for studies that compared the binding of monoclonal antibody 8H.8, or a polyclonal antibody raised to recombinant IgE (Re-hu IgE), to bind to a solid phase having peptide E3a.5 or substituted peptide E3a.5 (designated peptide S3a.5) immobilized thereon.
Fig. 16 depicts data from a study evaluating the ability of antibody 8H.8 conjugated to a signal moiety to bind to solid phases having various peptides immobilized thereon.
Fig. 17 depicts the ability of polyclonal anti-human IgE antibodies to bind to various peptides immobilized on a solid phase.

### MODES FOR CARRYING OUT INVENTION

### Proposed Mechanism of Action of Anti-IgE Monoclonal Antibodies in Causing Persistent Serum IgE Depletion

A hypothesis in accordance with the present invention is that anti-IgE monoclonal antibodies affect IgE levels by directly binding with the resident circulating IgE, after which the bound complex is removed from the circulation.

Moreover, it is hypothesized that anti-IgE monoclonal antibodies affect IgE levels by interfering with the production of new IgE by B-cells. Memory B-cells, cells that upon interacting with antigen and T-cell become antibody producing cells (i.e., "memory IgE B-cells"), are involved in replenishing serum IgE, and these cells contain IgE as their cell surface receptors. Thus, it is possible that anti-IgE monoclonal antibodies bind to the IgE on these antibody producing cells and interfere with their function, such as by down-regulation or by one or more mechanisms which lead to cell destruction. Two general ways have been proposed for how the binding of a monoclonal antibody to a memory B cell might interfere with production of IgE.

First, activation of a memory B cell, may also require the involvement of a surface IgE-binding factor designated CD23. Binding of certain monoclonal antibodies to the cell surface IgE may preclude CD23 binding, and may thus lead to an inability to mount an IgE response.

Second, IgE antibody production by a memory IgE B cell may be reduced or eliminated by binding of a monoclonal antibody that is directed against, or blocks, the receptor for the IgE molecule which is located on the surface of the memory B cell.

In the absence of a monoclonal antibody that leads to memory IgE B cell elimination or inactivation, however, it would be expected that the memory IgE B-cells would be replenished, leading to the eventual replenishment of circulating IgE.

The serum levels of IgE are elevated in patients experiencing allergic disease. As disclosed herein, when antibodies were generated that specifically bind to a species' IgE, and these antibodies were administered to a patient who is a member of the species ("passive immunization"), that patient's serum levels of IgE declined. As appreciated by one of ordinary skill in the art, the specific binding proteins in accordance with the invention are administered in pharmacologically accepted dosages, and can be administered with suitable biocompatible diluents. Also disclosed herein are a method and related compositions that serve to induce a response to a self-peptide, such as an IgE molecule, whereby the immune system is manipulated so as to allow an auto-reactive B cell to become an antibody-secreting B cell ("active immunization").

Receptors are present on mast cells that bind to IgE from the circulation. When IgE has become bound to the receptors on mast cells, the IgE molecules can become cross-linked. Upon cross-linking these IgE molecules, the mast cell is induced to release histamine. Histamine is an agent that induces the manifestation of allergic symptoms. Generally, cross-linking occurs by binding of the IgE molecules to an antigen, e.g., an allergen. However, a monoclonal antibody that binds to IgE could serve as a means for cross-linking IgE which has become bound to the surface of mast cells.

For allergic individuals, if anti-IgE antibodies were present in circulation, it becomes easy to envisage that the binding of such auto-anti-IgE to IgE molecules on mast cells could serve to cross-link the IgE molecules on those cells and exacerbate an ongoing allergic response even in the absence of allergen. This occurrence is clearly undesirable in the context of allergy treatment. An antibody that achieved this function would therefore not be preferred for use as a therapeutic in accordance with the invention. Therefore, an antibody that achieves such crosslinking is disadvantageous, and is not preferred in accordance with the invention.

Two approaches exist for producing monoclonal antibodies which target IgE, but which do not cross-link IgE molecules which have become bound to a mast cell. The first is to produce monoclonal antibodies that are directed to an epitope of an IgE molecule that is only accessible when the IgE is in circulation. Since the monoclonal antibody can only bind to IgE when it is in circulation, it will not be able to bind to IgE that has become bound to the surface of a mast cell.

An alternative approach for producing anti-IgE antibodies has been hypothesized by Stanworth et al, e.g., U.S. Patent No. 5,601,821 issued 11 February 1997. The antibodies of Stanworth are disclosed to cross-link IgE on mast cells, but in a manner that does not induce histamine release. Stanworth has disclosed an epitope of the human IgE molecule which must be available or accessible after IgE molecules have become cross-linked on the surface of a mast cell, in order for the mast cell to release histamine. Thus, Stanworth disclosed an antibody that binds to that particular epitope. Accordingly, monoclonal antibodies that are directed to this IgE epitope will serve to cross-link the IgE, but will not permit the mast cell to release histamine.

The approach followed in accordance with the present invention is the development of antibodies, either *ex vivo* monoclonal or *in vivo* anti-self antibodies, directed to epitopes which are accessible on circulating IgE but which are not accessible when such IgE becomes bound to a mast cell.

### Passive Immunity

### Monoclonal Antibodies 15A.2 and 8H.8

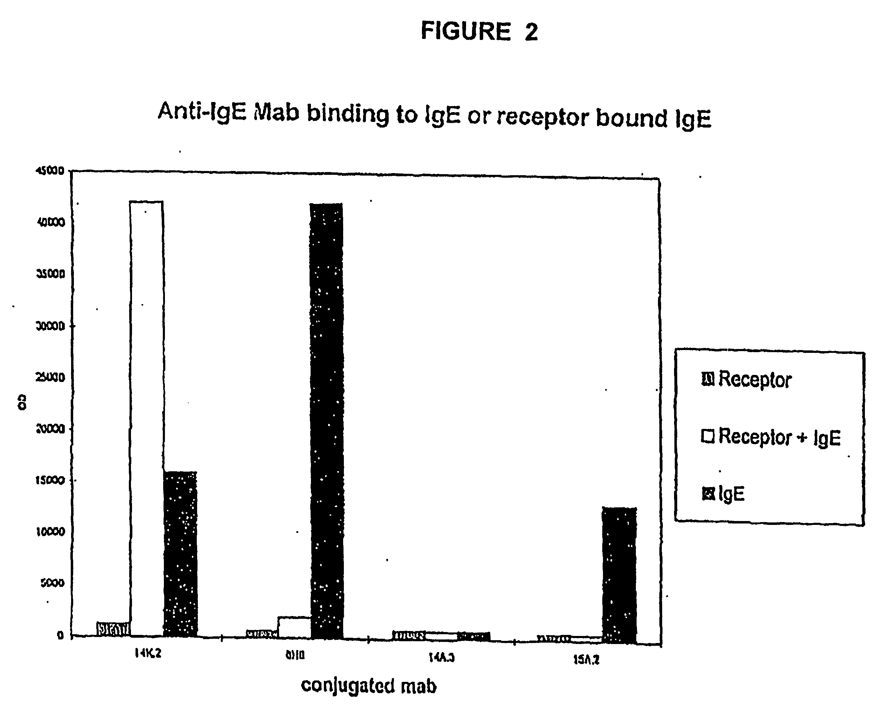

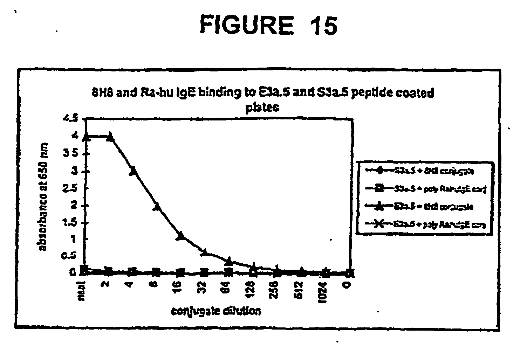

Monoclonal antibody 15A.2 binds to canine IgE. The 15A.2 monoclonal antibody was derived by immunizing mice with affinity-purified canine IgE. The epitope bound by 15A.2 is conformational, not linear. As indicated by data depicted in Figure 2, 15A.2 did not bind to the IgE receptor; nor did it bind to IgE when bound to receptor. However, 15A.2 exhibited affinity for free IgE. It binds to recombinant canine exon 3 fusion proteins in an enzyme linked immunosorbent assay (ELISA) and by Western blot, but not to other recombinant canine IgE fusion proteins.

Characterization experiments of the 15A.2 monoclonal antibody showed that 15A.2 did not bind to IgE that was already bound by the IgE receptor on mast cells. Thus, it appeared that access to the epitope bound by 15A.2 was hindered by IgE binding to the Fc receptor on mast cells. Accordingly, 15A.2 will not crosslink IgE bound to mast cells. This finding was demonstrated by binding studies with a recombinant receptor discussed herein.

Monoclonal antibody 8H.8 also binds to canine IgE. The 8H.8 monoclonal antibody was derived by immunizing mice with a shortened version of exon 3 of the canine IgE molecule, designated exon 3a. Exon 3a contains the C-terminal 71 amino acids of the full length exon 3. Previous studies (data not presented herein) had shown that immunizing mice with the full length exon 3 did not generate antibodies having specificity such as that ultimately found with the 8H.8 antibody.

Characterization experiments of the 8H.8 monoclonal antibody showed that, unlike monoclonal antibodies derived by immunization with all other recombinant IgE sequences, 8H.8 would also bind to native canine IgE. Additionally, like 15A.2 and as depicted in Figure 2, it was found that 8H.8 did not bind to IgE that was already bound by the IgE receptor on mast cells. Thus, it appeared that access to the epitope bound by 8H.8 was also hindered by IgE binding to the Fc receptor on mast cells. As a result, 8H.8 will not crosslink IgE bound to mast cells. This finding was demonstrated by binding studies with a recombinant receptor discussed herein.

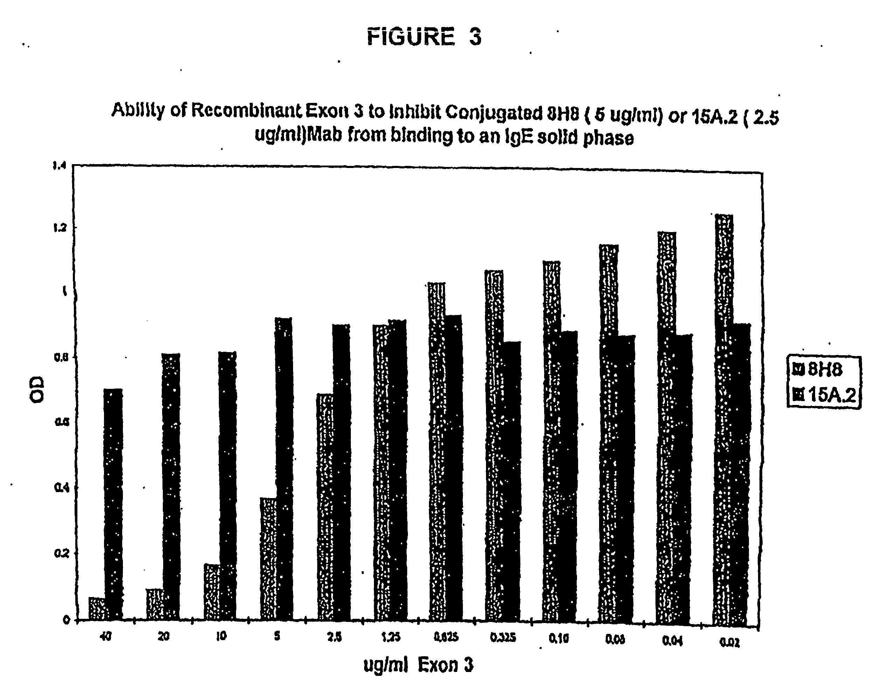

Competitive assays were performed between an antibody, 8H.8 or 15A.2, and soluble exon 3 to identify the level of inhibition of binding of these antibodies to affinity purified native IgE immobilized on the solid phase of an ELISA. The results from this study are depicted in Figure 3. In Figure 3 it is seen that increasing the concentration of recombinant exon 3 inhibited the binding of monoclonal antibody 8H.8, but did not inhibit antibody 15A.2.

In addition, the ability of these monoclonal antibodies to inhibit IgE from binding to recombinant IgE receptor on an ELISA solid phase was examined.

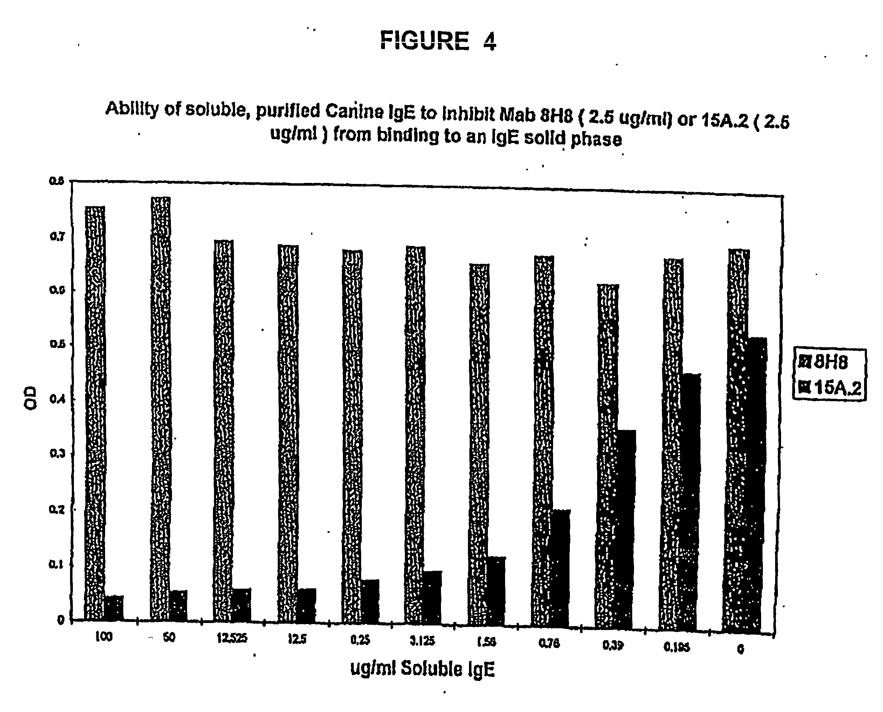

Additionally, competitive assays were performed with antibody 8H.8, and analogous assays were performed with 15A.2, where the antibody was in competition with affinity-purified canine IgE in solution and IgE on an ELISA solid phase. Data from these assays is depicted in Figure 4.

These studies suggested that 15A.2 had a high affinity for soluble native canine IgE because as the concentration of the soluble IgE was increased, the binding of 15A.2 to the immobilized immunoglobulin dropped appreciably, indicating that 15A.2 was now binding to the soluble IgE. In contrast, it is seen that antibody 8H.8 has a much lower affinity for soluble IgE, because increasingly high concentrations of the soluble IgE did not inhibit the ability of 8H.8 to bind to the immobilized IgE. Thus, the data suggest that soluble IgE was not effective at inhibiting 8H.8 from binding to IgE immobilized on a solid phase. Thus, the affinity of 8H.8 for soluble canine IgE was lower than the affinity of this monoclonal antibody for IgE on a solid phase.

These studies further suggested that 8H.8 had a low affinity for soluble native canine IgE, because increasingly high concentrations of the soluble IgE did not inhibit the ability of 8H.8 to bind to the immobilized IgE. In contrast, it is seen that antibody 15A.2 has a much higher affinity for soluble IgE, since as the concentration of the soluble IgE was increased, the binding of 15A.2 to the immobilized immunoglobulin dropped appreciably, indicating that 15A.2 was now binding to the soluble IgE. Thus, the data suggested that soluble IgE was not effective at inhibiting 8H.8 from binding to IgE immobilized on a solid phase. Thus, the affinity of 8H.8 for soluble canine IgE was lower than the affinity of this monoclonal for IgE on a solid phase.

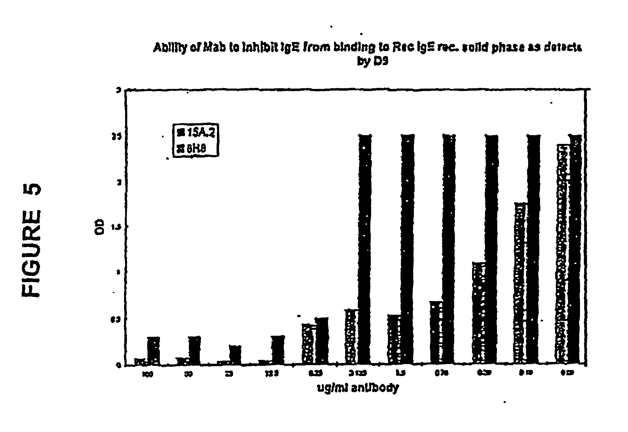

To further substantiate the finding that 15A.2 has a higher affinity for soluble native IgE, and that 8H.8 had a low affinity for soluble native IgE, studies were performed where 8H.8 was used, and analogous studies were conducted with 15A.2, to determine the extent to which each antibody inhibits binding of the soluble IgE to a recombinant IgE receptor solid phase. A labeled antibody D9 was used as a means for detection in the studies depicted in Figure 5. The monoclonal antibody D9 is known to bind to IgE when bound to receptor. For the studies depicted in Figure 5, the native IgE was present at a concentration of 2.5 micrograms per milliliter. As shown in Figure 5, 15A.2 was effective at inhibiting binding of the native IgE to recombinant IgE receptor at antibody concentrations as low as 190 nanograms per milliliter. In contrast, 3,125 nanograms per milliliter of 8H.8 were necessary to inhibit the binding of native IgE to the recombinant IgE receptor. Thus, since more 8H.8 than 15A.2 was required to inhibit the binding of native IgE to the recombinant receptor, it was seen that 8H.8 had a lower affinity for the soluble IgE. It appears, therefore, that when a sufficient concentration of either 8H.8 or 15A.2 antibodies is provided, that the antibodies impair the binding of soluble IgE to the IgE receptor.

Furthermore, as indicated by the data depicted in Figure 2, the avidity of 8H.8 binding for native canine IgE was greatly increased when the IgE was immobilized. These studies suggested that 8H.8 had a low affinity for soluble native canine IgE, but that when the IgE is immobilized on a surface, or when it is expressed on the surface of a memory B cell, the avidity of 8H.8 binding for native canine IgE was greatly increased.

Accordingly, in view of these findings, it was hypothesized that the region within native IgE recognized by 8H.8 might be partially hidden, particularly when the IgE is in serum. A partially sequestered amino acid sequence of IgE may not be readily exposed to the native canine immune system and its normal protective mechanisms. Furthermore, since the full length exon 3 did not lead to the generation of antibodies having 8H.8-like specificity, the full length sequence might contain suppressor peptides capable of down-regulating or eliminating an auto-anti-IgE response which is specific against the epitope recognized by the 8H.8 antibody. Either mechanism could serve to avoid production of an autoimmune response to self-antigens.

The implications of these findings are that 8H.8 antibody, when used as an allergy therapeutic in dogs, would preferentially bind to IgE on memory B cells rather than to IgE in solution. Moreover, 8H.8 has a low binding affinity for IgE when bound by Fc receptor on mast cells.

Phage display technology was used to map the epitope bound by the 8H.8 antibody. A preferred method for performing phage display technology is accomplished by use of a Ph.D. Phage Display Peptide Library Kit (New England BioLabs, Beverly Mass.). It was found that a 7 amino acid peptide (Thr-Leu-Leu-Glu-Tyr-Arg-Met) inhibited monoclonal antibody 8H.8 from competitive binding to either native or recombinant canine IgE. This sequence contained 6 amino acids common in form and/or spacing to the C-terminal region of exon 3. An 11 amino acid peptide synthesized from this region (Gly-Met-AsnLeu-Thr-Trp-Tyr-Arg-Glu-Ser-Lys) designated E3a.5 also inhibited monoclonal antibody 8H.8 from competitive binding to native or recombinant IgE.

It is believed that antibody responses with specificity such as that of 8H.8 do not occur naturally, even upon occurrence of events which would induce auto-anti-IgE responses to other epitopes, because the 8H.8 epitope is only partially available for recognition. Nevertheless, it is hypothesized that antibodies to an 8H.8-type epitope, generated from peptide immunization in accordance with the invention do bind to the partially available epitope, as does the 8H.8 monoclonal antibody.

It is further hypothesized that the region within native IgE recognized by 15A.2 might serve as an immunogen to induce auto-anti-IgE responses and might generate antibodies capable of binding to IgE+ B cells and of down regulating IgE synthesis. The implications of these findings are that 15A.2 antibody, when used as an allergy therapeutic in dogs, would prevent IgE from binding to mast cells and potentially affect the synthesis of IgE by B cells.

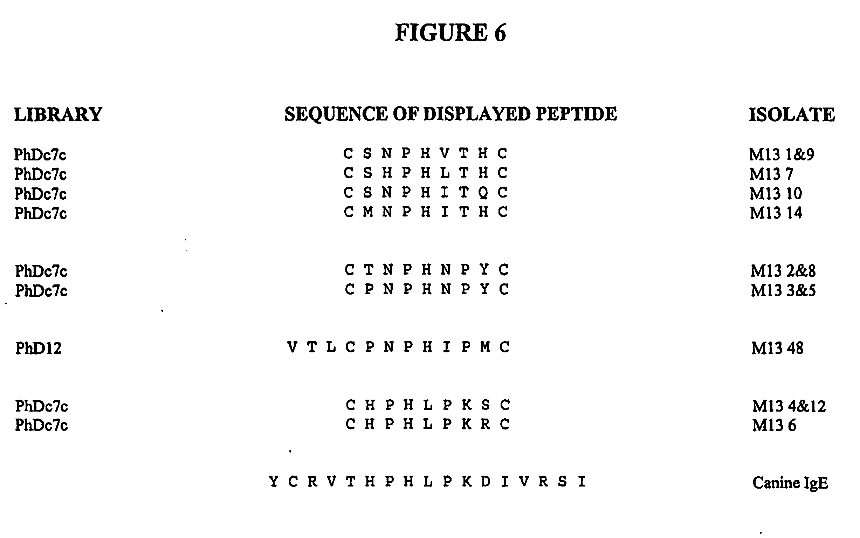

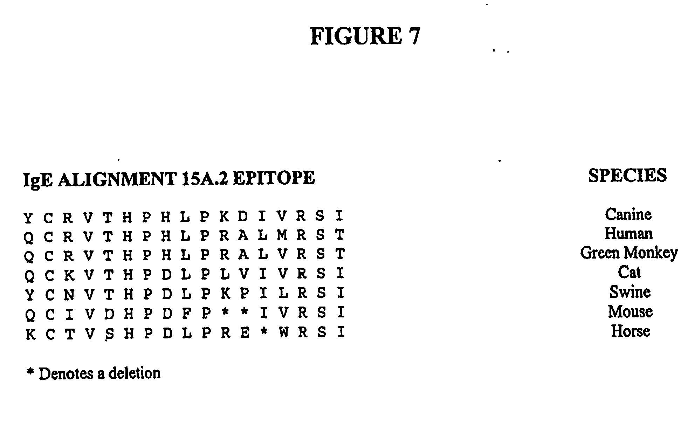

Phage display technology was used to map the epitope bound by the 15A.2 antibody. As noted above, a preferred method for performing phage display technology is accomplished by use of a Ph.D. Phage Display Peptide Library Kit (New England BioLabs, Beverly Mass.). Figure 6 depicts the amino acid sequences of the PhDc7c library that were bound by the monoclonal antibody 15A.2. These sequences are shown in alignment with the protein sequence of canine IgE. Figure 7 depicts the alignment of the 15A.2 epitope from seven different mammals: dog, human, green monkey, cat, swine, mouse and horse.

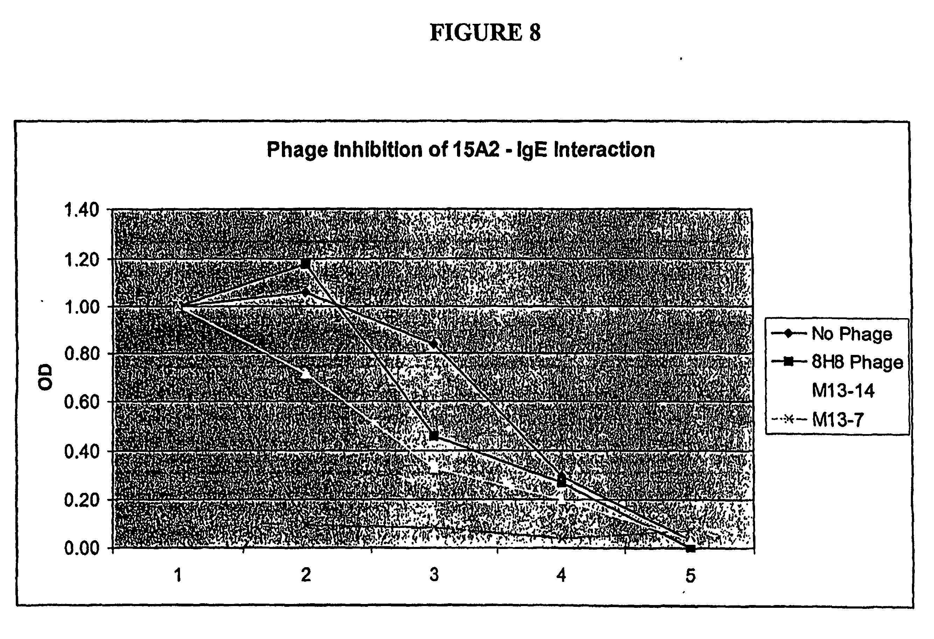

Figure 8 portrays the ability of different phage displaying 15A.2 mimotope peptides to inhibit canine IgE from binding the 15A.2 monoclonal antibody on the solid phase. 466 µl of biotinylated 15A.2 antibody (10 µg/ml) were mixed with 466 µl of phage from a fresh overnight culture. Into each well of a microtiter plate 100 µl of the mixture was added. The antibodies and phage were allowed to bind for 2 ½ hours. The wells were then coated with strepavidin. The plates were washed three times with standard wash buffer to remove loosely bound material. A serial dilution of canine IgE was prepared, starting with the concentration of 1 µg/100 µl of PBS, 0.1% Tween. 100 µl of dilution IgE was added per well and allowed to bind for 10 minutes. The competition reaction was stopped by washing the plates five times with wash solution. The plate was then developed with HRPO linked D9 monoclonal antibody, which binds to domain 4 of IgE, to visualize the IgE in the solid phase. Loss of signal indicates the phage successfully repelled canine IgE competition. Table 1 shows the concentrations of the reactants at the various points on the x-axis of figure 8.

**TABLE 1**

| | **IgE** | **phage** |
|---|---|---|
| 1 | 1.0 µg | 0 |
| 2 | 1.0 µg | 50 µl |
| 3 | 0.5 µg | 50 µl |
| 4 | 0.25 µg | 50 µl |
| 5 | 0.125 µg | 50 µl |

Using the New England Biolabs PhD12 phage display library, it was found that a 12 amino acid peptide (SEQ ID NO:11 Val-Thr-Leu-Cys-Pro-Asn-Pro-His-Ile-Pro-Met-Cys) inhibited monoclonal antibody 15A.2 from competitive binding to either native or recombinant canine IgE. This sequence contained 4 amino acids common in form and/or spacing to the N-terminal region of exon 3.

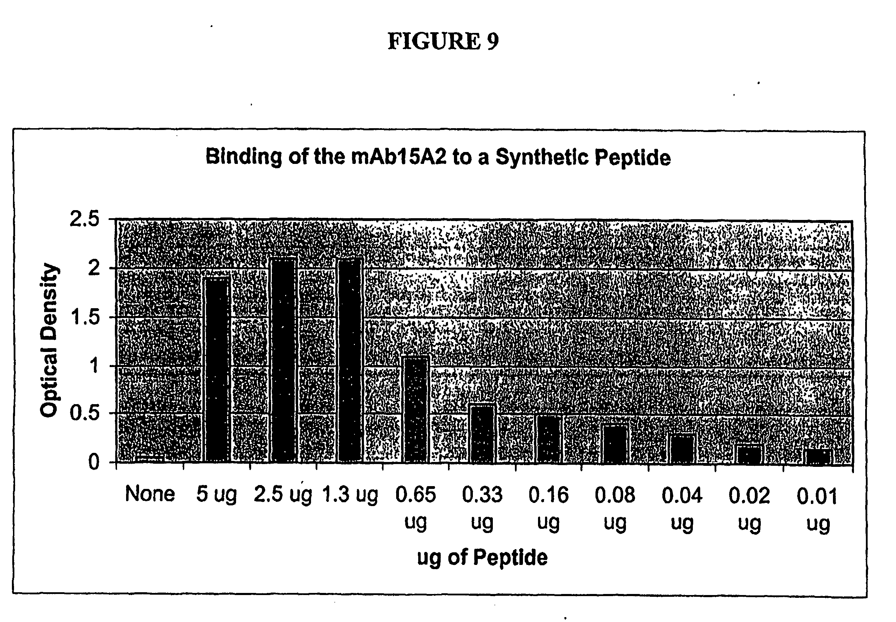

Figure 9 displays the ability of 15A.2 monoclonal antibody to bind a synthetic peptide. The peptide SEQ ID NO:12 Ser-Val-Thr-Leu-Cys-Pro-Asn-Pro-His-Ile-Pro-Met-Cys-Gly-Gly-Gly-Lys was synthesized and biotinylated on the epsilon carbon of the Lys residue. This sequence corresponds to the isolate M13-48. The peptide was treated in a manner to promote reduction and cyclization of the cysteines to form a cyclic peptide. A serial dilution of the peptide was prepared and bound to strepavidin-coated microtiter plates (5 µg strepavidin per well). Bound peptide was detected with HRPO conjugated 15A.2 monoclonal antibody. Figure 9 shows the results of this experiment. This solid phase specific 15A.2 mimotope peptide bound the 15A.2 raonloclonal antibody in a concentration dependent manner.

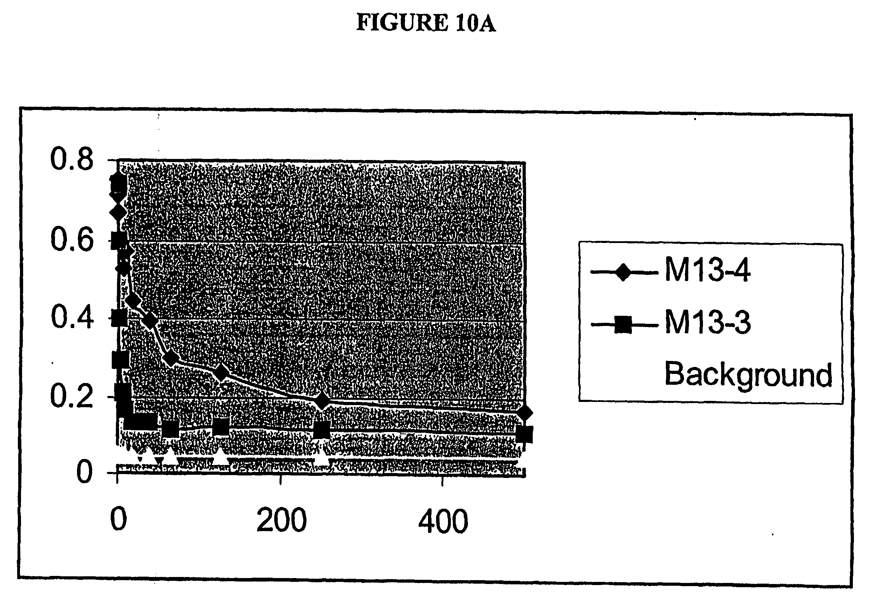

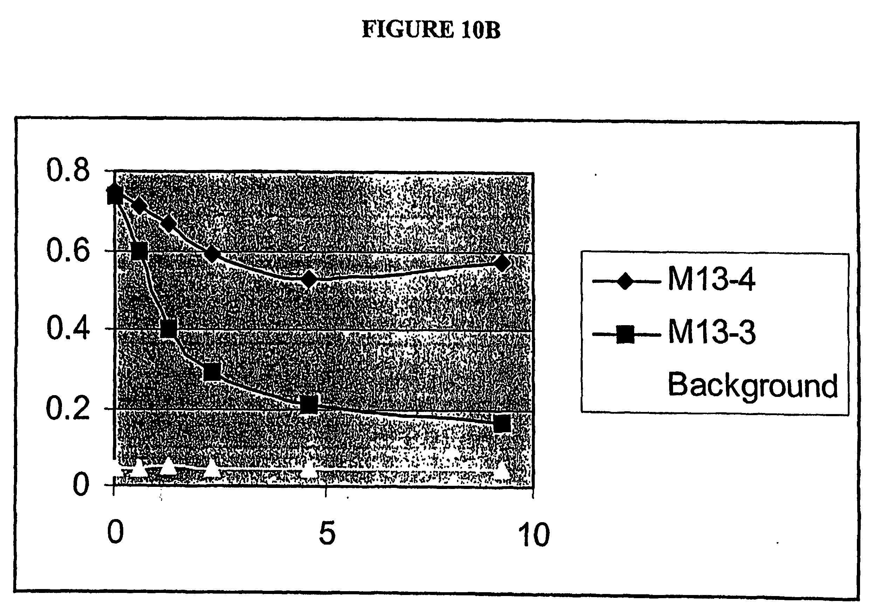

Figures 10A and 10B depict the ability of a 15A.2 mimotope peptide to prevent the 15A.2 monoclonal antibody from binding canine IgE on the solid phase. Plates were coated with canine IgE at a concentration of 1 µg/ml. A serial dilution of the synthetic peptide was added to HRPO conjugated 15A.2 monoclonal antibody (final concentration 1 µg/ml) and allowed to bind for two hours. 100 µl of the 15A.2/peptide mixture was added to the wells and allowed to bind 1 hour. The reaction was stopped by washing the plates six times with wash buffer. The plates were developed with HRPO substrate, the reactions stopped with stop mix, and the plates were measured for O.D. using a plate reader.

Apart from active immunization with antibody 15A.2 or 8H.8 to induce auto-anti-IgE production *in vivo*, this invention also comprises a specific binding molecule that specifically binds a ligand of the type bound by 15A.2 or 8H.8, as well as the therapeutic or prophylactic use thereof. Thus, the invention comprises a monoclonal antibody specific for a conservative variant of a sequence bound by 15A.2, for a conservative variant of a sequence bound by 8H.8, or for a native sequence of canine IgE up to 100 amino acids from the C' terminal portion of exon 3. The specific binding molecules of the invention can be used in a method in accordance with the invention as a treatment for or as prophylaxis for allergy symptoms, i.e., passive immunization.

Accordingly, administration to a dog of either an antibody in accordance with the invention, such as 15A.2 or 8H.8, or a peptide in accordance with the invention, such as the 7, 11 or 12 amino acid peptide, leads to a diminution of further IgE production. This may occur, for example, by the 15A.2 or 8H.8 antibody binding to the memory B cell and preventing further IgE production. For a peptide, its administration would lead to production of antibodies of comparable specificity and effect as 15A.2 or 8H.8.

### EXAMPLES

### Example 1

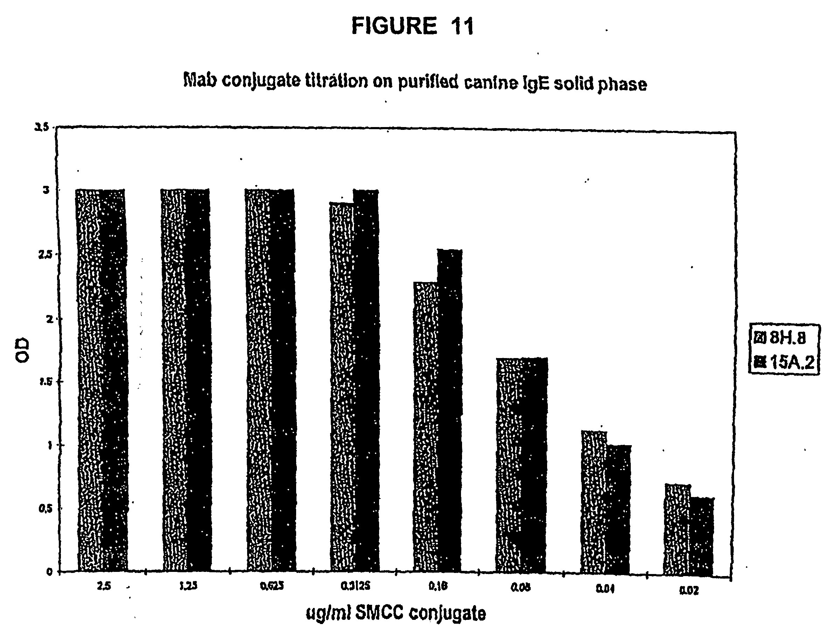

Figure 11 depicts results of the binding study where antibody 8H.8 and antibody 15A.2 were separately assayed to determine their ability to bind to a solid phase having affinity purified canine IgE immobilized thereon. These results showed that those 8H.8 and 15A.2 bind to native IgE when immobilized on a solid phase. The results from these studies suggest that the binding of each of these antibodies to the surface bound IgE was of comparable affinity. To determine whether the affinity of binding of each antibody was, in fact, comparable, further studies were performed.

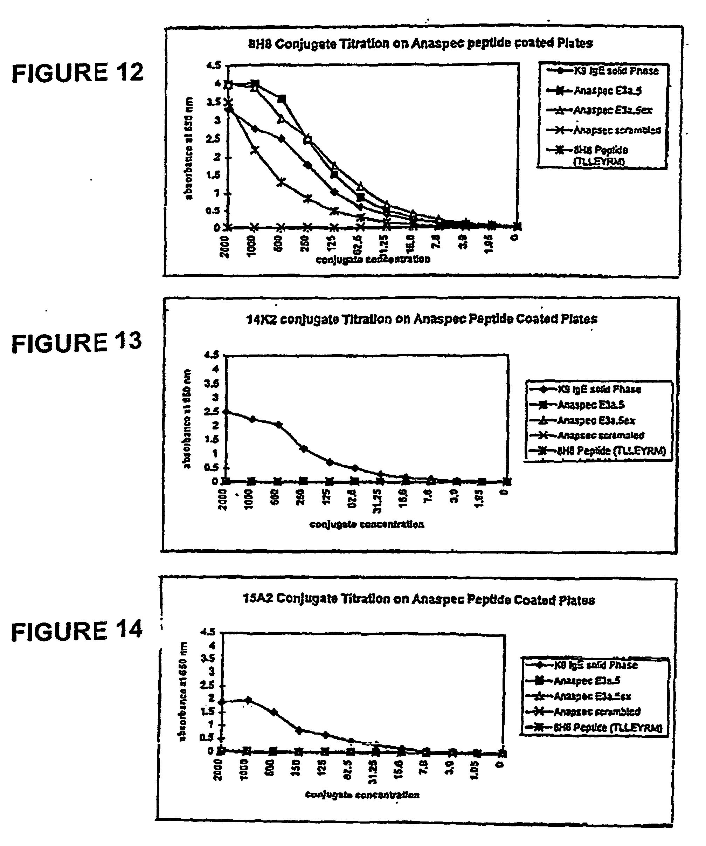

Figure 12 depicts results of the study where antibody 8H.8 conjugated to a signal moiety was reacted at various concentration with solid phases having various peptides immobilized thereon. The data depicted by (◆) reflects a solid phase having canine IgE immobilized thereon; data depicted by (■) depicts solid phase having E3a.5 immobilized thereon; data depicted by (π) depicts solid phase have E3a.5 extended peptide immobilized thereon, data depicted by an (X) reflects data for a solid phase having an E3a.5 scrambled peptide immobilized thereon, data depicted by (*) depicts data for solid phase having the seven amino acid peptide identified by phage display technology to which SH8 binds immobilized thereon. Thus, it is seen that 8H.8 binds to each solid phase with the exception solid phase having the E3a.5 scrambled peptide.

Monoclonal antibody 14K2 is known to bind exclusive to canine IgE exon 4. This monoclonal antibody was reacted with various solid phases having different peptides immobilized thereon. The data depicted by (◆) reflects a solid phase having canine IgE immobilized thereon; data depicted by (■) depicts solid phase having E3a.5 immobilized thereon; data depicted by (π) depicts solid phase have E3a.5 extended peptide immobilized thereon; data depicted by an (X) reflects data for a solid phase having an E3a.5 scrambled peptide immobilized thereon; and data depicted by (*) depicts data for solid phase having the seven amino acid peptide identified by phage display technology to which 8H.8 binds immobilized thereon. Thus, it is seen that 14K2 only binds to a solid phase having the entire canine IgE molecule immobilized thereon. This data is depicted in Figure 13.

Monoclonal antibody 15A.2 is believed to bind only to canine IgE exon 3. Figure 14 depicts data for studies which evaluated the binding of 15A.2 to various peptides. The data depicted by (◆) reflects a solid phase having canine IgE immobilized thereon; data depicted by (■) depicts solid phase having E3a.5 immobilized thereon; data depicted by (π) depicts solid phase have E3a.5 extended peptide immobilized thereon, data depicted by an (X) reflects data for a solid phase having an E3a.5 scrambled peptide immobilized thereon, data depicted by (*) depicts data for solid phase having the seven amino acid peptide identified by phage display technology to which 8H.8 binds immobilized thereon. These binding studies showed that 15A.2 bound only to a solid phase which had the entire canine IgE molecule immobilized thereon. This data indicated that 15A.2 does not bind the same epitope as that bound by 8H.8.

Figure 15 depicts data for studies that compare the binding of 8H.8 or polyclonal antibodies raised to recombinant human IgE, to peptide E3a.5 or to peptide E3a.5 with an amino acid substitution making the peptide more analogous to a sequence in the human genome which encodes human IgE, designated peptide S3a.5. In Figure 15 data depicted by the (◆) reflects substituted 3a.5 compared with 8H.8; data depicted by (■) reflects substituted peptide 3a.5 and Re-Hu IgE: data depicted by (n) reflects peptide E3a.5 and 8H.8 and, data depicted by and (X) reflects data regarding peptide E3a.5 and polyclonal Re Hu IgE. These data indicated that only 8H.8 became bound to E3a.5 on a solid phase, no other combination exhibited binding.

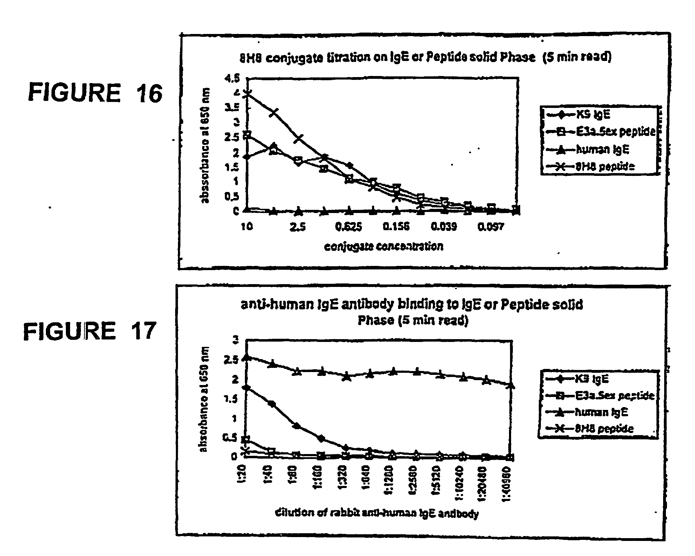

Figure 16 depicts the data on studies that compared the binding of 8H.8 to various peptides immobilized on a solid phase. Data depicted by (◆) reflects data for a solid phase having canine IgE immobilized thereon; data depicted by (■) reflects data for a solid phase having a peptide E3a.5 extended immobilized thereon; data depicted by (π) reflects data for a solid phase having human IgE immobilized thereon and, data depicted by an (X) reflects data for a solid phase having a 8H.8 peptide immobilized thereon. Accordingly it is seen that 8H.8 binds to canine IgE, peptide E3a.5 extended or the seven amino acid peptide identified by phage display technology as the binding epitope for 8H.8, when each of these peptides were immobilized on a solid phase. Additionally, it is seen that 8H.8 does not bind to human IgE.

Figure 17 depicts data for studies which compare the ability of polyclonal anti human IgE antibodies ability to bind to solid phases having various peptides immobilized thereon. Data depicted by (◆) reflects data for a solid phase having canine IgE immobilized thereon; data depicted by (■) reflects data for a solid phaae having a peptide E3a.5 extended immobilized thereon; data depicted by (π) reflects data for a solid phase having human IgE immobilized thereon and, data depicted by an (X) reflects data for a solid phase having a 8H.8 peptide immobilized thereon. Thus, it was seen that the anti-human IgE polyclonal antibodies bound to human IgE when immobilized on a solid phase. It was also seen that the polyclonal antiserum exhibited limited binding to peptide E3a.5 extended when the polyclonal antibodies were present at very high concentrations; this was believed to be a binding artifact due to the high antibody concentration. Thus, it was found that antibodies to Human IgE do not specifically bind to canine IgE or to peptides in accordance with the invention.

### Example 2

The amino acid sequences for the epitope bound by 8H.8, the 11 amino acid sequence used in the immunization studies presented herein, as well as analogous sequences from feline and Human IgE sequences are set forth in Table 2. Additionally, a substituted canine sequence was prepared having an amino acid substitution which made the peptide more closely analogous to the human peptide.

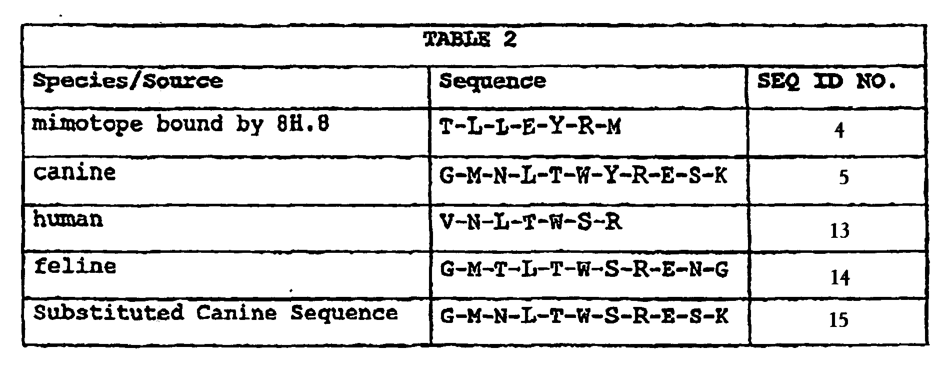

Each of the peptides in Table 2 was placed on a solid phase and studies were performed to identify if 8H.8 would bind to the surface-bound peptide. It was found that 8H.8 bound to a surface having the mimotope peptide thereon, and to a surface having the 11 amino acid peptide bound thereon. It was found that 8H.8 did not bind to the human peptide, the feline peptide or to the canine sequence which had an amino acid substitution which made it more analogous to the human sequence, when either of these peptides were surface bound.

### Closing

Unless defined otherwise, all technical and scientific terms used herein have the same meaning as commonly understood by one of ordinary skill in the art to which this invention belongs. Although any methods and materials similar to or equivalent to those described herein can be used in the practice or testing of the invention, the preferred methods and materials are now described.

## Claims

1. A specific binding protein selected from the group consisting of a monoclonal antibody, a polyclonal antibody, an antigen-binding fragment of a monoclonal antibody, an antigen binding fragment of a polyclonal antibody, a hybrid antibody, and a singe chain antibody, which specifically binds to native canine free or B cell-bound IgE by binding to an amino acid sequence comprising: (SEQ ID NO: 1) leucine-blank-blank-tyrosine-arginine; (SEQ ID NO: 2) tyrosine-arginine-blank-blank-leucine; 
(SEQ ID NO: 3) leucince-blank-blank-tyrosine-arginine-
blank-blank-leucine;
 
(SEQ ID NO: 4) threonine-leucine-leucine-glutamic acid-
tyrosine-arginine-metionine;
 
(SEQ ID NO: 5) glycine-methionine-asparagine-leucine-
threonine-tryptophan-tyrosine-arginine-glutamic acid-
serine-lysine;
 
(SEQ ID NO: 6) cysteine-blank-blank-proline-histidine-
blank-blank-blank-cysteine;
 
(SEQ ID NO: 7) serine-valine-threonine-leucine-cysteine-
proline-asparagine-proline-histidine-isoleucine-proline-
methionine-cysteine-glycine-glycine-glycine;
 
(SEQ ID NO: 8) serine-alanine-cysteine-proline-
asparagine-proline-histidine- asparagine-proline-tyrosine-
cysteine-glycine-glycine-glycine;
 
(SEQ ID NO: 9) cysteine-blank-proline-histidine-blank-
proline-blank-blank-cysteine;
 
(SEQ ID NO: 10) serine-alanine-cysteine-histidine-
proline-histidine-leucine-proline- lysine-serine-cysteine-
glycine-glycine-glycine;
 
(SEQ ID NO: 11) valine-threonine-leucine-cysteine-
proline-asparagine-proline-histidine-isoleucine-proline-
methionine-cysteine;
 or 
(SEQ ID NO: 12) serine-valine-threonine-leucine-cysteine-
proline-asparagine-proline-histidine-isoleucine-proline-
methionine-cysteine-glycine-glycine-glycine;
 where blank is any amino acid, and which does not bind to IgE when the IgE is bound to a receptor on a mast cell.

2. The specific binding protein of claim 1, wherein the B cell-bound IgE is IgE expressed on the surface of a canine B cell.

3. The specific binding protein of claim 1 or 2, which specifically binds to an isolated and purified peptide comprising a leucine positioned two peptide bonds away from a tyrosine-arginine pair.

4. The specific binding protein of claim 3, which specifically binds to a peptide comprising SEQ ID NO: 1, SEQ ID NO: 2 or SEQ ID NO: 3.

5. The specific binding protein of claim 4, wherein at least one blank is an amino acid with an aromatic ring.

6. The specific binding protein of any of claims 1 to 5, which is an antibody.

7. The antibody of claim 6, which is a monoclonal antibody.

8. The antibody of claim 6, which is raised to an isolated and purified peptide comprising a leucine positioned two peptide bonds away from a tyrosine-arginine pair, and wherein the peptide consists of from 5 to 71 amino acids.

9. The antibody of claim 8, which is raised to an isolated and purified peptide comprising an amino acid sequence, which comprises SEQ ID NO: 4, or a conservative variant thereof.

10. The antibody of claim 8, which is raised to an isolated and purified peptide comprising an amino acid sequence, which comprises SEQ ID NO: 5, or a conservative variant thereof.

11. The specific binding protein of any of claims 1 to 10, which is raised to a multiply antigenic peptide comprising multiple copies of an isolated and purified peptide which comprises a leucine positioned two peptide bonds away from a tyrosine-arginine pair.

12. The specific binding protein of claim 11, wherein said peptide consists of from 5 to 71 amino acids.

13. The specific binding protein of any of claims 1 to 12, which is raised to a recombinant plant virus particle comprising at least one copy of an isolated and purified peptide comprising a leucine positioned two peptide bonds away from a tyrosine-arginine pair.

14. The specific binding protein of claim 13, wherein said peptide consists of from 5 to 71 amino acids.

15. The specific binding protein of any of claims 1 to 14 that specifically binds to a defined epitope.

16. The specific binding protein of claim 15, which is a recombinant binding molecule.

17. The specific binding protein of claim 1, wherein the specific binding protein is a monoclonal antibody derived by immunizing mice with exon 3a of the canine IgE molecule, which specifically binds to free IgE or. B cell-bound IgE, and which does not bind to IgE when IgE is bound by the IgE receptor.

18. The specific binding protein of claim 1, wherein the specific binding protein is a monoclonal antibody derived by immunizing mice with affinity-purified canine IgE molecule, which specifically binds to free IgE or B cell-bound IgE, and which does not bind to IgE when IgE is bound by the IgE receptor.

19. The specific binding protein of any of claims 1 to 3, which specifically binds to an isolated and purified peptide comprising SEQ ID NO: 6, SEQ ID NO: 9 or SEQ ID NO: 12.

20. The specific binding protein of any of claims 1 to 3, which specifically binds to an isolated and purified peptide comprising SEQ ID NO: 13 (cysteine-proline-asparagine-proline-histidine-isoleucine-proline-methionine-cysteine) or SEQ ID NO: 14 (cysteine-proline-asparagine-proline-histidine-asparagine-proline-tyrosine-cysteine).

21. The specific binding protein of claim 20, wherein said peptide comprises the sequence serine-alanine-cysteine-proline-asparagine-proline-histidine-asparagine-proline-tyrosine-cysteine-glycine-glycine-glycine.

22. The specific binding protein of any of claims 19 to 21, which is an antibody.

23. The antibody of claim 22, which is a monoclonal antibody.

24. The antibody of claim 22, which is raised to an isolated and purified peptide comprising SEQ ID NO: 6, SEQ ID NO: 9 or SEQ ID NO: 12, or a conservative variant thereof.

25. The antibody of claim 22, which is raised to an isolated and purified peptide comprising SEQ ID NO: 13 or SEQ ID NO: 14, or a conservative variant thereof.

26. The antibody of claim 25, wherein said peptide comprises the sequence serine-alanine-cysteine-proline-asparagine-proline-histidine-asparagine-proline-tyrosine-cysteine-glycine-glycine-glycine.

27. The specific binding protein of any of claims 19 to 26 that specifically binds to a defined epitope.

28. The specific binding protein of claim 27, which is a recombinant binding molecule.

29. The use of a specific binding protein of any of claim 1 to 28 for the manufacture of a pharmaceutical composition for the treatment or prophylaxis of canine allergy.

30. The use of claim 29, wherein the pharmaceutical composition further comprises a diluent.

31. The use of claim 29 or 30 for the manufacture of a pharmaceutical composition kit, said kit comprising a first dose of said specific binding protein and a booster dose of said specific binding protein.

## Patentansprüche

1. Ein spezifisches Bindeprotein, das aus der Gruppe ausgewählt wird, die aus einem monoklonalen Antikörper, einem polyklonalen Antikörper, einem antigenbindenden Fragment eines monoklonalen Antikörpers, einem antigenbindenden Fragment eines polyklonalen Antikörpers, einem Hybrid-Antikörper und einem einkettigen Antikörper besteht, das dadurch spezifisch an natives, freies oder an B-Zellen gebundenes Hunde-IgE bindet, dass es an eine Aminosäuresequenz bindet, wobei die Aminosäuresequenz umfasst: 
(SEQ ID NO: 1) Leucin-Platzhalter-Platzhalter-Tyrosin-
Arginin;
 
(SEQ ID NO: 2) Tyrosin-Arginin-Platzhalter-Platzhalter-
Leucin;
 
(SEQ ID NO: 3) Leucin-Platzhalter-Platzhalter-Tyrosin-
Arginin-Platzhalter-Platzhalter-Leucin;
 
(SEQ ID NO: 4) Threonin-Leucin-Leucin-Glutaminsäure-Tyrosin-
Arginin-Methionin;
 
(SEQ ID NO: 5) Glycin-Methionin-Asparagin-Leucin-Threonin-
Tryptophan-Tyrosin-Arginin-Glutaminsäure-Serin-Lysin;
 
(SEQ ID NO: 6) Cystein-Platzhalter-Platzhalter-Prolin-
Histidin-Platzhalter-Platzhalter-Platzhalter-Cystein;
 
(SEQ ID NO: 7) Serin-Valin-Threonin-Leucin-Cystein-Prolin-
Asparagin-Prolin-Histidin-Isoleucin-Prolin-Methionin-Cystein-
Glycin-Glycin-Glycin;
 
(SEQ ID NO: 8) Serin-Alanin-Cystein-Prolin-Asparagin-Prolin-
Histidin-Asparagin-Prolin-Tyrosin-Cystein-Glycin-Glycin-
Glycin;
 
(SEQ ID NO: 9) Cystein-Platzhalter-Prolin-Histidin-
Platzhalter-Prolin-Platzhalter-Platzhalter-Cystein;
 
(SEQ ID NO: 10) Serin-Alanin-Cystein-Histidin-Prolin-
Histidin-Leucin-Prolin-Lysin-Serin-Cystein-Glycin-Glycin-
Glycin;
 
(SEQ ID NO: 11) Valin-Threonin-Leucin-Cystein-Prolin-
Asparagin-Prolin-Histidin-Isoleucin-Prolin-Methionin-Cystein;
 oder 
(SEQ ID NO: 12) Serin-Valin-Threonin-Leucin-Cystein-Prolin-
Asparagin-Prolin-Histidin-Isoleucin-Prolin-Methionin-Cystein-
Glycin-Glycin-Glycin;
 wobei der Platzhalter jede Aminosäure sein kann, und das nicht an IgE bindet, wenn das IgE an einen Rezeptor auf einer Mastzelle gebunden ist.

2. Das spezifische Bindeprotein gemäß Anspruch 1, wobei das B-Zell-gebundene IgE ein auf der Oberfläche einer B-Zelle gebundenes Hunde-IgE ist.

3. Das spezifische Bindeprotein gemäß Anspruch 1 oder 2, das an ein isoliertes oder gereinigtes Peptid, das ein zwei Peptidbindungen entfernt von einem Tyrosin-Arginin-Paar angeordnetes Leucin umfasst, spezifisch bindet.

4. Das spezifische Bindeprotein gemäß Anspruch 3, das an ein Peptid, das SEQ ID NO: 1, SEQ ID NO: 2 oder SEQ ID NO: 3 umfasst, spezifisch bindet.

5. Das spezifische Bindeprotein gemäß Anspruch 4, wobei mindestens ein Platzhalter eine Aminosäure mit einem aromatischen Ring ist.

6. Das spezifische Bindeprotein gemäß einem der Ansprüche 1 bis 5, das ein Antikörper ist.

7. Der Antikörper gemäß Anspruch 6, der ein monoklonaler Antikörper ist.

8. Der Antikörper gemäß Anspruch 6, der gegen ein isoliertes und gereinigtes Peptid, das ein zwei Peptidbindungen entfernt von einem Tyrosin-Arginin-Paar angeordnetes Leucin umfasst, gerichtet ist, wobei das Peptid aus 5 bis 71 Aminosäuren besteht.

9. Der Antikörper gemäß Anspruch 8, der gegen ein isoliertes und gereinigtes Peptid, das eine Aminosäuresequenz, welche die SEQ ID NO: 4 oder eine konservative Variante davon umfasst, umfasst, gerichtet ist.

10. Der Antikörper gemäß Anspruch 8, der gegen ein isoliertes und gereinigtes Peptid, das eine Aminosäuresequenz, welche die SEQ ID No: 5 oder eine konservative Variante davon umfasst, umfasst, gerichtet ist.

11. Das spezifische Bindeprotein gemäß einem der Ansprüche 1 bis 10, das gegen ein mehrfach antigenisches Peptid, das mehrere Kopien eines isolierten und gereinigten Peptids, das ein zwei Peptidbindungen entfernt von einem Tyrosion-Arginin-Paar angeordnetes Leucin umfasst, umfasst, gerichtet ist.

12. Das spezifische Bindeprotein gemäß Anspruch 11, wobei das Peptid aus 5 bis 71 Aminosäuren besteht.

13. Das spezifische Bindeprotein gemäß einem der Ansprüche 1 bis 12, das gegen ein rekombinantes Pflanzenviruspartikel, das mindestens eine Kopie eines isolierten und gereinigten Peptids, das ein zwei Peptidbindungen entfernt von einem Tyrosin-Arginin-Paar angeordnetes Leucin umfasst, umfasst, gerichtet ist.

14. Das spezifische Bindeprotein gemäß Anspruch 13, wobei das Peptid aus 5 bis 71 Aminosäuren besteht.

15. Das spezifische Bindeprotein gemäß einem der Ansprüche 1 bis 14, das mit einem definierten Epitop spezifisch bindet.

16. Das spezifische Bindeprotein gemäß Anspruch 15, das ein rekombinantes Bindemolekül ist.

17. Das spezifische Bindeprotein gemäß Anspruch 1, wobei das spezifische Bindeprotein ein monoklonaler Antikörper ist, der durch Immunisieren von Mäusen mit Exon 3a des Hunde-IgE-Moleküls, das an freies IgE oder B-Zell-gebundenes IgE spezifisch bindet und das nicht an IgE bindet, wenn IgE durch den IgE-Rezeptor gebunden ist, abgeleitet wird.

18. Das spezifische Bindeprotein gemäß Anspruch 1, wobei das spezifische Bindeprotein ein monoklonaler Antikörper ist, der durch Immunisierung von Mäusen mit einem Affinitätsgereinigten Hunde-IgE-Molekül, das an freies IgE oder B-Zell-gebundenes IgE spezifisch bindet und das nicht an IgE bindet, wenn IgE durch den IgE-Rezeptor gebunden ist, abgeleitet wird.

19. Das spezifische Bindeprotein gemäß einem der Ansprüche 1 bis 3, das an ein isoliertes und gereinigtes Peptid, das die SEQ ID NO: 6, SEQ ID NO: 9 oder SEQ ID NO: 12 umfasst, spezifisch bindet.

20. Das spezifische Bindeprotein gemäß einem der Ansprüche 1 bis 3, das an ein isoliertes und gereinigtes Peptid, das die SEQ ID NO: 13 (Cystein-Prolin-Asparagin-Prolin-Histidin-Isoleucin-Prolin-Methionin-Cystein) oder SEQ ID NO: 14 (Cystein-Prolin-Asparagin-Prolin-Histidin-Asparagin-Prolin-Tyrosin-Cystein) umfasst, spezifisch bindet.

21. Das spezifische Bindeprotein gemäß Anspruch 20, wobei das Peptid die Sequenz Serin-Alanin-Cystein-Prolin-Asparagin-Prolin-Histidin-Asparagin-Prolin-Tyrosin-Cystein-Glycin-Glycin-Glycin umfasst.

22. Das spezifische Bindeprotein gemäß einem der Ansprüche 19 bis 21, das ein Antikörper ist.

23. Der Antikörper gemäß Anspruch 22, der ein monoklonaler Antikörper ist.

24. Der Antikörper gemäß Anspruch 22, der gegen ein isoliertes und gereinigtes Peptid, das die SEQ ID NO: 6, SEQ ID NO: 9 oder SEQ ID NO: 12 oder eine konservative Variante davon umfasst, gerichtet ist.

25. Der Antikörper gemäß Anspruch 22, der gegen ein isoliertes und gereinigtes Peptid, das die SEQ ID NO: 13 oder SEQ ID NO: 14 oder eine konservative Variante davon umfasst, gerichtet ist.

26. Der Antikörper gemäß Anspruch 25, wobei das Peptid die Sequenz Serin-Alanin-Cystein-Prolin-Asparagin-Prolin-Histidin-Asparagin-Prolin-Tyrosin-Cystein-Glycin-Glycin-Glycin umfasst.

27. Das spezifische Bindeprotein gemäß einem der Ansprüche 19 bis 26, das an ein definiertes Epitop spezifisch bindet.

28. Das spezifische Bindeprotein gemäß Anspruch 27, das ein rekombinantes Bindemolekül ist.

29. Die Verwendung eines spezifischen Bindeproteins gemäß einem der Ansprüche 1 bis 28 für die Herstellung einer pharmazeutischen Zusammensetzung zur Behandlung oder Prophylaxe einer Allergie gegen Hunde.

30. Die Verwendung gemäß Anspruch 29, wobei die pharmazeutische Zusammensetzung des Weiteren ein Verdünnungsmittel umfasst.

31. Die Verwendung gemäß Anspruch 29 oder 30 für die Herstellung eines Kits einer pharmazeutischen Zusammensetzung, wobei das Kit eine erste Dosis des spezifischen Bindeproteins und eine Boosterdosis des spezifischen Bindeproteins umfasst.

## Revendications

1. Protéine de liaison spécifique choisie au sein du groupe constitué d'un anticorps monoclonal, un anticorps polyclonal, un fragment de liaison à l'antigène d'un anticorps monoclonal, un fragment de liaison à l'antigène d'un anticorps polyclonal, un anticorps hybride et un anticorps monocaténaire, qui se lie spécifiquement à une IgE canine libre ou liée à un lymphocyte B par liaison à une séquence d'acides aminés comprenant : la (SEQ ID N°1) leucine-blanc-blanc-tyrosine-arginine ; la (SEQ ID N°2) tyrosine-arginine –blanc-blanc-leucine; 
la (SEQ ID N°3) leucine-blanc-blanc-tyrosine-arginine-blanc-blanc-
leucine ;
 
la (SEQ ID N°4) thréonine-leucine-leucine-acide glutamique-tyrosine-
arginine-méthionine ;
 
la (SEQ ID N°5) glycine-méthionine-asparagine-leucine-thréonine-
tryptophane-tyrosine-arginine-acide glutamique-sérine-lysine ;
 
la (SEQ ID N°6) cystéine- blanc-blanc-proline-histidine-blanc-blanc-
blanc-cystéine ;
 
la (SEQ ID N°7) sérine-valine-thréonine-leucine-cystéine-proline-
asparagine-proline-histidine-isoleucine-proline-méthionine-cystéine-
glycine-glycine-glycine ;
 
la (SEQ ID N°8) sérine-alanine-cystéine-proline-asparagine-proline-
histidine-asparagine-proline-tyrosine-cystéine-glycine-glycine-glycine ;
 
la (SEQ ID N°9) cystéine-blanc-proline-histidine-blanc-proline-blanc-
blanc-cystéine ;
 
la (SEQ ID N°10) sérine-alanine-cystéine-histidine-proline-histidine-
leucine-proline-lysine-sérine-cystéine-glycine-glycine-glycine ;
 
la (SEQ ID N°11) valine-thréonine-leucine-cystéine-proline-asparagine-
proline-histidine-isoleucine-proline-méthionine-cystéine ;
 ou 
la (SEQ ID N°12) sérine-valine-thréonine-leucine-cystéine-proline-
asparagine-proline-histidine-isoleucine-proline-méthionine-cystéine-
glycine-glycine-glycine ;
 où « blanc » signifie un acide aminé quelconque, et qui ne se lie pas à l'IgE quand l'IgE est liée à un récepteur présent sur un mastocyte.

2. Protéine de liaison spécifique selon la revendication 1, où l'IgE liée au lymphocyte B est une IgE exprimée à la surface d'un lymphocyte B canin.

3. Protéine de liaison spécifique selon la revendication 1 ou 2, qui se lie spécifiquement à un peptide isolé et purifié comprenant une leucine située à une distance de deux liaisons peptidiques d'un couple tyrosine-arginine.

4. Protéine de liaison spécifique selon la revendication 3, qui se lie spécifiquement à un peptide comprenant la SEQ ID N° 1, la SEQ ID N° 2 ou la SEQ ID N°3.

5. Protéine de liaison spécifique selon la revendication 4, où au moins un blanc est un acide aminé possédant un noyau aromatique.

6. Protéine de liaison spécifique selon l'une quelconque des revendications 1 à 5, qui est un anticorps.

7. Anticorps selon la revendication 6 qui est un anticorps monoclonal.

8. Anticorps selon la revendication 6 qui est dirigé contre un peptide isolé et purifié comprenant une leucine située à une distance de deux liaisons peptidiques d'un couple tyrosine-arginine, et où le peptide est constitué de 5 à 71 acides aminés.

9. Anticorps selon la revendication 8 qui est dirigé contre un peptide isolé et purifié comprenant une séquence d'acides aminés qui comprend la SEQ ID N°4 ou un variant conservatif de celle-ci.

10. Anticorps selon la revendication 8 qui est dirigé contre un peptide isolé et purifié comprenant une séquence d'acides aminés qui comprend la SEQ ID N°5 ou un variant conservatif de celle-ci.

11. Protéine de liaison spécifique selon l'une quelconque des revendications 1 à 10, dirigée contre une multiplicité de peptides antigéniques comprenant des exemplaires multiples d'un peptide isolé et purifié qui comprend une leucine située à une distance de deux liaisons peptidiques d'un couple tyrosine-arginine.

12. Protéine de liaison spécifique selon la revendication 11, où ledit peptide est constitué des acides aminés 5 à 71.

13. Protéine de liaison spécifique selon l'une quelconque des revendications 1 à 12, dirigée contre une particule virale végétale recombinante comprenant au moins un exemplaire d'un peptide isolé et purifié qui comprend une leucine située à une distance de deux liaisons peptidiques d'un couple tyrosine-arginine.

14. Protéine de liaison spécifique selon la revendication 13, où ledit peptide est constitué des acides aminés 5 à 71.

15. Protéine de liaison spécifique selon l'une quelconque des revendications 1 à 14, qui se lie spécifiquement à un épitope défini.

16. Protéine de liaison spécifique selon la revendication 15, qui est une molécule de liaison recombinante.

17. Protéine de liaison spécifique selon la revendication 1, où la protéine de liaison spécifique est un anticorps monoclonal obtenu en immunisant des souris avec l'exon 3a de la molécule d'IgE canine, qui se lie spécifiquement à des IgE libres ou liées à des lymphocytes B et qui ne se lie pas à une IgE quand l'IgE est liée au récepteur de l'IgE.

18. Protéine de liaison spécifique selon la revendication 1, où la protéine de liaison spécifique est un anticorps monoclonal obtenu en immunisant des souris avec une molécule d'IgE canine purifiée par affinité, qui se lie spécifiquement à des IgE libres ou liés à des lymphocytes B et qui ne se lie pas à une IgE quand l'IgE est liée au récepteur de l'IgE.

19. Protéine de liaison spécifique selon l'une quelconque des revendications 1 à 3 qui se lie spécifiquement à un peptide isolé et purifié comprenant la SEQ ID N° 6, la SEQ ID N° 9 ou la SEQ ID N°12.

20. Protéine de liaison spécifique selon l'une quelconque des revendications 1 à 3 qui se lie spécifiquement à un peptide isolé et purifié comprenant la SEQ ID N° 13 (cystéine-proline-asparagine-proline-histidine-isoleucine-proline-méthionine-cystéine) ou la SEQ ID N° 14 (cystéine-proline-asparagine-proline-histidine-asparagine-proline-tyrosine-cystéine).

21. Protéine de liaison spécifique selon la revendication 20, où ledit peptide comprend la séquence sérine-alanine-cystéine-proline-asparagine-proline-histidine-asparagine-proline-tyrosine-cystéine-glycine-glycine-glycine.

22. Protéine de liaison spécifique selon l'une quelconque des revendications 19 à 21 qui est un anticorps.

23. Anticorps selon la revendication 22 qui est un anticorps monoclonal.

24. Anticorps selon la revendication 22, qui est dirigé contre un peptide isolé et purifié comprenant la SEQ ID N° 6, la SEQ ID N° 9 ou la SEQ ID N° 12 ou un variant conservatif de celles-ci.

25. Anticorps selon la revendication 22, qui est dirigé contre un peptide isolé et purifié comprenant la SEQ ID N° 13 ou la SEQ ID N° 14 ou un variant conservatif de celles-ci.

26. Anticorps selon la revendication 25, où ledit peptide comprend la séquence sérine-alanine-cystéine-proline-asparagine-proline-histidine-asparagine-proline-tyrosine-cystéine-glycine-glycine-glycine.

27. Protéine de liaison spécifique selon l'une quelconque des revendications 19 à 26 qui se lie spécifiquement à un épitope défini.

28. Protéine de liaison spécifique selon la revendication 27 qui est une molécule de liaison recombinante.

29. Utilisation d'une protéine de liaison spécifique selon l'une quelconque des revendications 1 à 28 pour la fabrication d'une composition pharmaceutique destinée au traitement ou à la prophylaxie des allergies canines.

30. Utilisation selon la revendication 29, où la composition pharmaceutique comprend en outre un diluant.

31. Utilisation selon la revendication 29 ou 30 pour la fabrication d'un kit de compositions pharmaceutiques, ledit kit comprenant une première dose de ladite protéine de liaison spécifique et une dose de rappel de ladite protéine de liaison spécifique.
